# FASCICULE DE BREVET EUROPEEN

(11) **EP 2 303 808 B1**
(45) Date de publication et mention de la délivrance du brevet: **21.09.2016**
(21) Numéro de dépôt: 09737102.5
(22) Date de dépôt: 21.07.2009
(51) Int. Cl.: C07C 1/24, C07C 11/04, C07C 51/235, C07C 51/377, C07C 53/122, C07C 57/04, C07C 67/055, C07C 69/01, C07C 69/24, C08F 18/08, C08F 18/10, C12P 7/06, C12P 7/42

(54) **FABRICATION DE PROPIONATE DE VINYLE A PARTIR DE MATIERES RENOUVELABLES**
HERSTELLUNG VON PROPIONSÄUREVINYLESTER AUS ERNEUERBAREN MATERIALIEN
PRODUCTION OF VINYL PROPIONATE FROM RENEWABLE MATERIALS

(30) Priorité: 22.07.2008 FR 0854976
(43) Date de publication de la demande: 06.04.2011
(73) Titulaire: Arkema France, 92700 Colombes (FR)
(72) Inventeur: DUBOIS, Jean-Luc, F-69390 Millery (FR)
(74) Mandataire: Gavin, Pablo
(86) Numéro de dépôt international: PCT/FR2009/051462
(87) Numéro de publication internationale: WO 2010/010291

(56) Documents cités:
- EP-A- 1 710 227
- WO-A-2008/067627
- DE-A1- 10 225 339
- GB-A- 1 142 250
- GÜNTER ROSCHER: "Ullmann's Encyclopedia of Industrial Chemistry - Vinyl Esters" [Online] 15 juin 2000 (2000-06-15), WILEY-VCH VERLAG GMBH & CO KGAA , WEINHEIM , XP002519625 Extrait de l'Internet: URL:http://mrw.interscience.wiley.com/emrw /9783527306732/ueic/article/a27_419/curren t/pdf DOI: 10.1002/14356007.a27_419> alinéa [2.2.3] alinéas [02.4], [03.3]
- KLAUSMEIER, WILLIAM H.: "Biomass chemicals production by thermochemical conversion" BIOTECHNOLOGY AND BIOENGINEERING SYMPOSIUM , VOLUME DATE 1983, 13(SYMP. BIOTECHNOL. FUELS CHEM., 5TH, 1983), 81-97 CODEN: BIBSBR; ISSN: 0572-6565, 1984, XP002519624

## Description

La présente invention se rapporte à un procédé de fabrication de propionate de vinyle à partir d'alcools issus de la fermentation de matières premières renouvelables, de préférence les matières premières renouvelables sont des matières végétales.

Les esters de vinyle, et en particulier, le propionate de vinyle sont connus pour leurs utilisations comme monomère dans des (co)polymères, généralement par émulsion. Le propionate de vinyle permet la synthèse d'homopolymères ou de copolymères avec le chlorure de vinyle ou avec des acrylates d'alkyle comme par exemple l'acrylate de t-butyle.

Un des problèmes posés par les procédés de synthèse des esters de vinyle de l'art antérieur est qu'ils sont réalisés à partir de matières premières d'origine fossile (pétrole) non renouvelables, notamment de l'éthylène. Or les ressources en ces matières premières sont limitées, l'extraction du pétrole requiert d'aller creuser de plus en plus profond et dans des conditions techniques toujours plus difficiles nécessitant des équipements sophistiqués et la mise en oeuvre de procédés toujours plus coûteux en énergie. Ces contraintes ont une conséquence directe sur le coût de fabrication de l'éthylène et donc sur le coût de fabrication des esters de vinyle.

GB 1 142 250 décrit un procédé de fabrication de propionate de vinyle par acyloxylation de l'éthylène.

De manière avantageuse et surprenante, les inventeurs de la présente demande ont mis en oeuvre un procédé de fabrication industriel de propionate de vinyle à partir de matières premières renouvelables.

Le procédé selon l'invention permet de s'affranchir au moins en partie des matières premières d'origine fossile et de les remplacer par des matières premières renouvelables.

Le propionate de vinyle obtenu suivant le procédé selon l'invention est de qualité telle qu'il peut être utilisé dans toutes les applications dans lesquelles il est connu d'utiliser le propionate de vinyle, y compris dans les applications les plus exigeantes.

Il est donc décrit ici le propionate de vinyle dans lequel au moins une partie des atomes de carbone est d'origine renouvelable, cette partie d'origine renouvelable pouvant être déterminée selon la norme ASTM D 6866-06.

Les atomes de carbone d'origine renouvelable sont également appelés atomes de carbone contemporain ou bio ressourcé.

Il est également décrit une composition comprenant du propionate de vinyle et notamment une composition, plus particulièrement une solution, comprenant au moins 80%, de préférence au moins 90 % en poids de propionate de vinyle par rapport au poids total de la composition, les atomes de carbone dudit propionate de vinyle étant au moins en partie des atomes de carbone d'origine renouvelable.

Une solution de propionate de vinyle aussi concentrée n'a jamais été obtenue avec les procédés de synthèse d'esters de vinyle renouvelable de l'art antérieur.

L'invention a pour objet un procédé de fabrication du propionate de vinyle qui comprend une étape d'acyloxylation d'éthylène par l'acide propanoïque dans lequel au moins une partie des atomes de carbone de l'éthylène et/ou de l'acide propanoïque est issue d'origine renouvelable.

Plus précisément, l'invention a pour objet un procédé de fabrication de propionate de vinyle comprenant les étapes suivantes :
a) fermentation de matières premières renouvelables et, éventuellement purification pour produire au moins un alcool choisi parmi l'éthanol et les mélanges d'alcools comprenant de l'éthanol ;
b) déshydratation de l'alcool obtenu pour produire, dans un premier réacteur, au moins un alcène choisi parmi l'éthylène et les mélanges d'alcènes comprenant de l'éthylène et, éventuellement purification de l'éthylène ;
c) production d'acide acrylique à partir de matières premières renouvelables,
d) hydrogénation de l'acide acrylique en présence d'hydrogène moléculaire pour produire de l'acide propanoïque,
e) introduction dans un troisième réacteur de l'éthylène obtenu à l'issue de l'étape b) et de l'acide propanoïque obtenu à l'issus de l'étape d), et mise en oeuvre de la réaction de acyloxylation de l'éthylène,
f) isolation et éventuellement purification du propionate de vinyle obtenu à l'issue de l'étape e).

L'invention a également pour objet le propionate de vinyle susceptible d'être obtenu par le procédé selon l'invention, ou plus généralement le propionate de vinyle obtenu au moins en partie à partir de matières premières renouvelables.

Il est également décrit les utilisations du propionate de vinyle obtenu à partir de matières d'origine renouvelable ou d'une composition contenant au moins 80 % de propionate de vinyle obtenu à partir de matières d'origine renouvelable et en particulier les utilisations dudit propionate de vinyle ou de ladite composition pour la fabrication d'homopolymères ou de copolymères avec le chlorure de vinyle ou avec des acrylates d'alkyle comme par exemple l'acrylate de t-butyle.

D'autres objets, aspects, caractéristiques de l'invention apparaîtront à la lecture de la description suivante.

L'étape a) du procédé de fabrication de propionate de vinyle selon l'invention comprend la fermentation de matières premières renouvelables pour produire au moins un alcool, ledit alcool étant choisi parmi l'éthanol et les mélanges d'alcools comprenant de l'éthanol.

Une matière première renouvelable est une ressource naturelle, par exemple animale ou végétale, dont le stock peut se reconstituer sur une période courte à l'échelle humaine. Il faut en particulier que ce stock puisse se renouveler aussi vite qu'il est consommé. Par exemple, les matières végétales présentent l'avantage de pouvoir être cultivées sans que leur consommation aboutisse à une diminution apparente des ressources naturelles.

A la différence des matériaux issus de matières fossiles, les matières premières renouvelables contiennent du ¹⁴C . Tous les échantillons de carbone tirés d'organismes vivants (animaux ou végétaux) sont en fait un mélange de 3 isotopes : ¹²C (représentant environ 98,892 %), ¹³C (environ 1,108 %) et ¹⁴C (traces: 1,2.10⁻¹⁰ %)_{.} Le rapport ¹⁴C/¹²C des tissus vivants est identique à celui de l'atmosphère. Dans l'environnement, le ¹⁴C existe sous deux formes prépondérantes : sous forme de gaz carbonique (CO₂), et sous forme organique, c'est-à-dire de carbone intégré dans des molécules organiques.

Dans un organisme vivant, le rapport ¹⁴C/¹²C est maintenu constant par le métabolisme car le carbone est continuellement échangé avec l'environnement extérieur. La proportion de ¹⁴C étant constante dans l'atmosphère, il en est de même dans l'organisme, tant qu'il est vivant, puisqu'il absorbe ce ¹⁴C au même titre que le ¹²C ambiant. Le rapport moyen de ¹⁴C/¹²C est égal à 1,2×10⁻¹².

Le ¹²C est stable, c'est-à-dire que le nombre d'atomes de ¹²C dans un échantillon donné est constant au cours du temps. Le ¹⁴C est radioactif, le nombre d'atomes de ¹⁴C dans un échantillon décroît au cours du temps (t), sa demi-vie étant égale à 5730 ans.

La teneur en ¹⁴C est sensiblement constante depuis l'extraction des matières premières renouvelables, jusqu'à la fabrication du propionate de vinyle selon l'invention et même jusqu'à la fin de l'utilisation de l'objet comprenant du propionate de vinyle.

Par conséquent, la présence de ¹⁴C dans un matériau, et ce, quelle qu'en soit la quantité, donne une indication sur l'origine des molécules le constituant, à savoir qu'elles proviennent de matières premières renouvelables et non de matériaux fossiles.

La quantité de ¹⁴C dans un matériau peut être déterminée par l'une des méthodes décrites dans la norme ASTM D6866-06 (Standard Test Methods for Determining the Biobased Content of Natural Range Materials Using Radiocarbon and Isotope Ratio Mass Spectrometry Analysis).

Cette norme comporte trois méthodes de mesure du carbone organique issu de matières premières renouvelables, dénommé en langue anglaise « biobased carbon ». Les proportions indiquées pour l'ester de vinyle de l'invention sont de préférence mesurées selon la méthode par spectrométrie de masse ou la méthode par spectrométrie à scintillation liquide décrite dans cette norme, et tout préférentiellement par spectrométrie de masse.

Ces méthodes de mesure évaluent le rapport des isotopes ¹⁴C/¹²C dans l'échantillon et le comparent à un rapport des isotopes ¹⁴C/¹²C dans un matériau d'origine biologique donnant le 100% standard, afin de mesurer le pourcentage de carbone organique de l'échantillon.

De préférence, le propionate de vinyle selon l'invention comprend une quantité de carbone issu de matières premières renouvelables supérieure à 20%, de préférence supérieure à 40% en masse par rapport à la masse totale de carbone du propionate de vinyle.

En d'autres termes, le propionate de vinyle peut comporter au moins 0,25 10⁻¹⁰ % en masse de ¹⁴C, et de préférence au moins 0,5 10⁻¹⁰% en masse ¹⁴C.

Avantageusement la quantité de carbone issu de matières premières renouvelables est supérieure à 75%, de préférence égale à 100% en masse par rapport à la masse totale de carbone du propionate de vinyle.

En tant que matières premières renouvelables, on pourra utiliser des matières d'origine végétale ; des matières d'origine animale ou des matières d'origine végétale ou animale issues de matériaux récupérés (matériaux recyclés).

Au sens de l'invention, les matières végétales contiennent au moins des sucres et/ou amidons.

Les matières végétales contenant des sucres sont essentiellement la canne à sucre et la betterave sucrière, on peut également citer l'érable, le palmier-dattier, le palmier à sucre, le sorgho, l'agave américain ; les matières végétales contenant des amidons sont essentiellement les céréales et légumineuses comme le maïs, le blé, l'orge, le froment, le riz, la pomme de terre, le manioc, la patate douce, ou encore les algues.

Parmi les matières issues de matériaux récupérés, on peut notamment citer les déchets végétaux ou organiques comprenant des sucres et/ou amidons.

De préférence les matières premières renouvelables sont des matières végétales.

La fermentation des matières renouvelables s'effectue en présence d'un ou plusieurs microorganismes adéquats, ce microorganisme peut éventuellement avoir été modifié naturellement par une contrainte chimique ou physique, ou génétiquement, on parle alors de mutant. Classiquement le microorganisme utilisé est *Saccharomyces cerevisiae* ou un de ses mutants.

En tant que matières premières renouvelables, on peut également utiliser de la cellulose ou de l'hémicellulose voire de la lignine qui, en présence des microorganismes adéquats, peuvent être transformées en matières comprenant du sucre. Parmi ces matières renouvelables, on compte la paille, le bois, le papier qui peuvent provenir avantageusement de matériaux récupérés.

Les listes présentées ci-dessus ne sont pas limitatives.

De préférence, l'étape de fermentation (a) est suivie d'une étape de purification destinée à séparer l'éthanol des autres produits. Par exemple, on peut réaliser une étape de distillation pour séparer l'éthanol des autres produits.

A l'étape b) du procédé de fabrication du propionate de vinyle, est mise en oeuvre la déshydratation du ou des alcools obtenus à l'étape a) pour produire, dans un premier réacteur, au moins un alcène choisi parmi l'éthylène et les mélanges d'alcènes comprenant de l'éthylène, le produit secondaire de la déshydratation étant de l'eau.

Généralement, la déshydratation de l'alcool est effectuée à l'aide d'un catalyseur à base d'alumine comme le catalyseur commercialisé par EUROSUPPORT sous la dénomination commerciale ESM 110 ® (alumine trilobique non dopée contenant peu -environ 0,04%- de Na₂O résiduel). De préférence l'alumine est une gamma-alumine.

Les conditions opératoires de la déshydratation font partie des connaissances générales de l'homme du métier, à titre indicatif, la déshydratation est généralement effectuée à une température de l'ordre de 400 °C.

Un autre avantage du procédé selon l'invention est son économie en énergie: les étapes de fermentation et déshydratation du procédé selon l'invention sont effectuées à des températures relativement basses, inférieures à 500°C, de préférence inférieures à 400°C, en comparaison l'étape de craquage et de vapocraquage du pétrole en éthylène s'effectue à une température de l'ordre de 800°C.

Cette économie d'énergie s'accompagne aussi d'une diminution du taux de CO₂ émis dans l'atmosphère.

L'étape c) du procédé de fabrication du propionate de vinyle consiste en la fabrication d'acide acrylique à partir de matières premières renouvelables.

Selon une première variante, cette étape c) est effectuée à partir de glycérol issu de la méthanolyse des huiles végétales c'est-à-dire d'une matière d'origine renouvelable.

A l'étape c) de cette première variante, le glycérol, avantageusement sous forme d'une solution aqueuse de concentration comprise entre 10 et 50 % en poids, est soumis soit à une réaction de déshydratation du glycérol pour former l'acroléine puis une réaction d'oxydation de l'acroléine formée en présence d'oxygène moléculaire pour produire l'acide acrylique, soit à une réaction d' oxydéshydratation du glycérol en présence d'oxygène moléculaire pour former l'acide acrylique.

Ces réactions de déshydratation et d'oxydation sont connues de l'homme du métier : par exemple, dans le cas où on réalise la déshydratation puis l'oxydation en deux étapes distinctes, on peut utiliser l'enseignement du document EP1710227.

Dans le cas où on fait une oxydéshydratation en une étape, dans un même réacteur, dans un premier temps le glycérol est déshydraté en acroléine puis, en présence d'oxygène moléculaire, l'acroléine est oxydée en acide acrylique ; cette oxydéshydratation est notamment décrite dans la demande FR 2 884 817 de la Société Arkema SA. La réaction d'oxydéshydratation est généralement effectuée en présence d'un mélange de catalyseurs, et plus particulièrement d'un mélange d'un catalyseur solide acide (adapté pour la réaction de déshydratation) et d'un catalyseur d'oxydation tels que les catalyseurs solides contenant au moins un élément choisi dans la liste Mo, V, W, Re, Cr, Mn, Fe, Co, Ni, Cu, Zn, Sn, Te, Sb, Bi, Pt, Pd, Ru, Rh présent sous la forme métallique ou sous forme d'oxyde, de sulfate, ou de phosphate (adapté pour la réaction d'oxydation). La réaction est de préférence mise en oeuvre en phase gazeuse à une température comprise entre 250°C et 350°C et une pression comprise entre 1 et 5 bar.

Selon une seconde variante de l'étape c), on obtient l'acide acrylique par fermentation de matières premières renouvelables, pour produire de l'acide 3-hydroxypropionique, éventuellement purification, suivie d'une déshydratation de l'acide 3-hydroxypropionique en acide acrylique, éventuellement en présence d'oxygène moléculaire.

On peut utiliser comme matière première renouvelable les matières premières comprenant du sucre, de l'amidon, de la cellulose ou de l'hémicellulose utilisées lors de l'étape (a). Préférentiellement, les matières premières comprennent le glucose.

La fermentation des matières renouvelables s'effectue en présence d'un ou plusieurs microorganismes adéquats, ce microorganisme peut éventuellement avoir été modifié naturellement par une contrainte chimique ou physique, ou génétiquement, on parle alors de mutant. Classiquement le microorganisme utilisé est parmi *Escherichia coli* ou un de leurs mutants. Cette fermentation est connue de l'homme du métier et est décrite dans les demandes de brevet internationales WO 02/42418 et WO 2008/089102.

La déshydratation de l'acide 3-hydroxypropionique en acide acrylique peut être réalisée, en phase liquide ou en phase gazeuse, par un catalyseur de déshydratation.

Ces catalyseurs pourront généralement être constitués par un sel d'hétéropolyacide dans lequel des protons dudit hétéropolyacide sont échangés avec au moins un cation choisi parmi les éléments appartenant aux Groupes I à XVI de la Classification Périodique des Eléments, ces sels d'hétéropolyacide contenant au moins un élément choisi parmi le groupe comprenant W, Mo et V. Parmi les oxydes mixtes, on peut citer particulièrement ceux à base de fer et de phosphore et ceux à base de césium, phosphore et tungstène. Les catalyseurs sont notamment choisis parmi les zéolithes, les composites Nafion® (à base d'acide sulfonique de polymères fluorés), les alumines chlorées, les acides et sels d'acides phosphotungstiques et/ou silicotungstiques, et différents solides de type oxydes métalliques tels que oxyde de tantale Ta205, oxyde de niobium Nb205, alumine A12O3, oxyde de titane TiO2, zircone ZrO2, oxyde d'étain SnO2, silice SiO2 ou silico-aluminate SiO2-AI2O3, imprégnés de fonctions acides telles que borate B03, sulfate SO4, tungstate WO3, phosphate PO4, silicate SiO2, ou molybdate MoO3, ou un mélange de ces composés. Les catalyseurs précédents peuvent comprendre en plus un promoteur tel que Au, Ag, Cu, Pt, Rh, Pd, Ru, Sm, Ce, Yt, Sc, La, Zn, Mg, Fe, Co, Ni, or montmorillonite.

Les catalyseurs préférés sont les zircones phosphatées, les zircones tungstées, les zircones silicées, les oxydes de titane ou d'étain imprégnés de tungstate ou phosphotungstate, les alumines ou silices phosphatées, les hétéropolyacides ou sels d'hétéropolyacides, les phosphates de fer et les phosphates de fer comprenant un promoteur.

On peut réaliser cette déshydratation à une température de 350 à 400°C, éventuellement en présence d'oxygène moléculaire. La présence d'oxygène moléculaire permet d'augmenter la durée de vie du catalyseur lors de la réaction.

On peut également utiliser la déshydratation sont connues de l'homme du métier et sont décrites dans les exemples 17 à 26 de la demande de brevet internationale WO 02/090312.

On peut également se référer au rapport « Process Economics Program Report 259 - Chemicals from biobased C3S » de Ronald Bray (SRI Consulting) pour réaliser cette étape c).

A l'étape d) est effectuée l'hydrogénation de l'acide acrylique en présence d'hydrogène moléculaire pour obtenir de l'acide propanoïque.

Cette hydrogénation sélective peut être mise en oeuvre selon deux procédés principaux :
- par catalyse homogène en phase liquide, le catalyseur étant un complexe ruthénium-phosphine et le solvant étant le méthanol, à une température d'environ 60°C et à une pression d'environ 3MPa ;
- par catalyse hétérogène sur un catalyseur cuivre-zinc déposé sur un oxyde d'aluminium, la réaction est alors effectuée en lit fixe à une température comprise entre 250°C et 350°C et à une pression comprise entre 1 atm et environ 6 atm.

L'étape e) d'acyloxylation de l'éthylène est généralement effectuée en phase gazeuse, dans un réacteur à lit fixe, au moyen d'un catalyseur au palladium ou à l'acétate de palladium sur un support qui peut être du SiO₂ ou du A1₂O₃, à une température allant généralement de 175 à 200°C.

On peut ajouter des co-catalyseurs par exemple à base d'or, de rhodium, de platine ou de cadmium.

On peut aussi ajouter des acétates de métaux alcalins en vue d'améliorer la sélectivité et l'activité des catalyseurs.

De préférence au moins une étape de purification est effectuée lors de l'étape de fermentation et/ou lors de déshydratation de l'alcool et/ou lors des étapes de production de l'acide.

Les étapes éventuelles de purification (purification de(s) alcool(s) obtenus à l'étape a), purification de(s) alcène(s) obtenus à l'étape b), purification des acides obtenus aux étapes c) et d)) sont avantageusement conduites par absorption sur des filtres classiques tels que tamis moléculaires, zéolithes, noir de carbone...), ou encore par distillation des produits obtenus aux étapes a), b) c)ou d).

Si l'alcool obtenu à l'étape a) a été purifié de façon à isoler l'éthanol, l'alcène obtenu à l'étape b) est de l'éthylène.

Si l'alcool obtenu à l'étape a) n'a pas été purifié, on obtient à l'issue de l'étape b) un mélange d'alcènes comprenant de l'éthylène.

Avantageusement, on effectue au moins une étape de purification lors de l'étape a) et/ou de l'étape b) afin d'obtenir de l'éthylène de degré de pureté suffisant pour réagir avec l'acide propanoïque. On préférera obtenir de l'éthylène de degré de pureté supérieure à 85 % en poids, de préférence supérieure à 95 % en poids et de manière plus préférée 99 % en poids.

De manière particulièrement préférée, l'alcool obtenu à l'étape a) est purifié de façon à isoler l'éthanol, en conséquence l'alcène obtenu à l'étape b) est l'éthylène.

Les principales impuretés présentes dans l'éthylène issu de la déshydratation de l'éthanol sont l'éthanol, le propane et l'acétaldéhyde.

Avantageusement, l'éthylène devra être purifié, c'est-à-dire que l'éthanol, le propane et l'acétaldéhyde devront être éliminés, pour pouvoir réaliser facilement l'étape e) d'acyloxylation.

L'éthylène, l'éthanol, le propane et l'acétaldéhyde peuvent être séparés en mettant en oeuvre une ou plusieurs distillations à basse température.

Les températures d'ébullition de ces composés sont les suivantes :

| composé | température d'ébullition (°C) |
|---|---|
| éthylène | -103,7 |
| propane | -42,1 |
| acétaldéhyde | 20,8 |
| éthanol | 75,5 |

L'éthylène, l'éthanol, le propane et l'acétaldéhyde sont refroidis à environ -105°C, de préférence -103,7°C puis distillés pour extraire l'éthylène.

Un autre avantage du procédé selon la présente invention concerne les impuretés. Les impuretés présentes dans l'éthylène issu de la déshydratation de l'éthanol sont totalement différentes de celles présentes dans l'éthylène issu de vapocraquage. En particulier parmi les impuretés présentes dans l'éthylène issu de vapocraquage, on compte le dihydrogène et le méthane et ceci quelle que soit la composition de la charge initiale.

Classiquement la séparation du dihydrogène et du méthane s'effectue après compression à 36 bar et refroidissement à environ -120°C. Dans ces conditions le dihydrogène et le méthane, liquides, sont séparés dans le déméthaniseur ; puis l'éthylène est récupéré à 19 bar et -33°C.

Le procédé selon la présente demande permet de s'affranchir de l'étape de séparation du dihydrogène et du méthane, et permet également de refroidir le mélange à -105°C à pression atmosphérique au lieu de -120°C à 36 bar. Le refroidissement de cette étape de séparation peut se faire aussi sous pression pour augmenter la température d'ébullition des composés à séparer (par exemple vers 20 bar et -35°C). Ces différences contribuent également à rendre le procédé selon l'invention plus économique (économie de matériel et économie d'énergie qui s'accompagne aussi d'une diminution du taux de CO₂ émis dans l'atmosphère).

Un autre avantage est que l'éthylène obtenu à l'étape b) du procédé selon l'invention ne comprend pas d'acétylène contrairement à l'éthylène obtenu par craquage ou vapocraquage. Or l'acétylène est très réactif et provoque des réactions secondaires d'oligomérisation, l'obtention d'éthylène sans acétylène est donc particulièrement avantageuse.

Un autre avantage est que le procédé selon l'invention peut être mis en oeuvre dans des unités de production localisées sur le lieu de production des matières premières. En outre, la taille des unités de production du procédé selon l'invention est beaucoup plus petite que la taille d'une raffinerie : les raffineries sont en effet de grosses installations situées généralement loin des centres de production des matières premières et alimentées par des pipelines.

Avantageusement, on effectue au moins une étape de purification lors de l'étape c) et/ou de l'étape d) afin d'obtenir de l'acide propanoïque de degré de pureté suffisant pour réagir avec l'éthylène. On préférera obtenir de l'acide propanoïque de degré de pureté supérieure à 85 % en poids, de préférence supérieure à 95 % en poids et de manière plus préférée 99 % en poids.

Le procédé de fabrication de propionate de vinyle selon l'invention est illustré par l'exemple suivant.

### EXEMPLE

### Fabrication d'éthylène

Dans cette installation, de l'éthanol à 96% obtenu par fermentation de glucose est vaporisé dans un vaporiseur, puis préchauffé dans un échangeur thermique, avant d'être injecté en tête d'un réacteur de 127 mm de diamètre contenant un lit catalytique porté à 300-400°C et constitué d'une couche d'alumine ESM110^{®} d'EUROSUPPORT, représentant un volume de 12700 cm³ et une masse de 6500 g, le rapport du débit volumique d'éthanol au volume de catalyseur étant de 1h⁻¹. Le mélange d'eau et d'éthylène produit dans le réacteur est refroidi dans l'échangeur thermique, avant d'être envoyé vers un séparateur gaz-liquide où l'éthylène et l'eau mélangé à des sous-produits sont séparés.

### Fabrication d'acide propionique à partir de glycérol

L'étape préliminaire consiste à purifier le glycérol brut obtenu à partir d'huile végétale, en éliminant les sels. Puis, on réalise la réaction de déshydratation du glycérol en acroléine et la condensation d'une partie de l'eau. La réaction de déshydratation est effectuée en phase gaz dans un réacteur en lit fixe en présence d'un catalyseur solide constitué par une zircone tungstée ZrO₂-WO; à une température de 320°C, sous pression atmosphérique. Un mélange de glycérol (20% massique) et eau (80% massique) est envoyé dans un vaporiseur, en présence d'air, dans un ratio molaire O₂ / glycérol de 0,6 / 1. Le milieu gazeux sortant du vaporiseur à 290°C est introduit dans le réacteur, constitué d'un tube de diamètre 30mm chargé de 390ml de catalyseur, plongé dans un bain de sel (mélange eutectique KNO₃, NaNO₃, NaNO₂) maintenu à la température de 320°C. En sortie du réacteur, le mélange réactionnel gazeux est envoyé en pied d'une colonne de condensation.

Dans l'étape suivante, le mélange gazeux est introduit, après addition d'air (ratio molaire O₂ / acroléine de 0,8 / 1) et d'azote en quantité nécessaire pour obtenir une concentration d'acroléine de 6,5% molaire, en alimentation du réacteur d'oxydation de l'acroléine en acide acrylique. Ce réacteur d'oxydation est constitué d'un tube de diamètre 30mm chargé de 480ml d'un catalyseur commercial d'oxydation d'acroléine en acide acrylique à base d'oxydes mixtes d'aluminium, molybdène, silicium, vanadium et cuivre plongé dans un bain de sel identique à celui décrit ci-dessus , maintenu lui à une température de 345°C. Avant introduction sur le lit catalytique, le mélange gazeux est préchauffé dans un tube plongeant également dans le bain de sel.

Après passage dans une colonne d'absorption, on récupère une solution d'acide acrylique, d'eau et autres impuretés.

La solution aqueuse obtenue est soumise à une étape de séchage par une distillation pour éliminer l'eau sous forme d'un mélange azéotropique avec la méthylisobutylcétone (MIBK). Une solution de stabilisants dans la MIBK est injectée en continu en tête de colonne, contenant les stabilisants hydroquinone, phénothiazine et dibutyldithiocarbamate de butyle (respectivement : 35ppm, 70ppm, 35ppm par rapport à l'acide acrylique contenu dans le flux d'alimentation). Le mélange azéotropique distille à une température de tête de 45°C sous une pression de 1,2.10⁴ Pa.

L'acide acrylique séché, récupéré en pied de colonne, ne contient plus que 0,4% d'eau et d'autres impuretés.

Un évaporateur tubulaire à double paroi en acier inoxydable (longueur du tube 100 cm, diamètre intérieur 2,5 cm, épaisseur de paroi 4 mm) a été garni sur toute sa longueur d'anneaux de Raschig en silice.

Un réacteur tubulaire à double paroi en acier inoxydable identique à l'évaporateur a été garni de bas en haut, d'abord sur une longueur de 5 cm, d'anneaux de Raschig, puis le réacteur tubulaire à double paroi a été garni d'un mélange homogène de 130 ml = 135,1 g du catalyseur d'hydrogénation Jonhson Mattey de type 50B (Pd à 0,3 % en poids sur gamma-A1₂O₃, en sphères de 2 mm) et de 226 ml d'anneaux de Raschig. Le reste de la longueur du réacteur tubulaire à double paroi n'a été garni que d'anneaux de Raschig.

Tant l'espace intermédiaire de l'évaporateur tubulaire à double paroi que celui du réacteur tubulaire à double paroi ont été garnis d'une huile formant un fluide caloporteur, qui présente une température de 185°C.

On a introduit la solution d'acide acrylique obtenue (de haut en bas) dans l'évaporateur tubulaire à double paroi avec un débit correspondant à 8,5 g/h d'acide acrylique introduit. A contre-courant de ces eaux-mères, on a fait passer par l'évaporateur tubulaire 16 moles/h d'hydrogène moléculaire.

Le mélange d'acide acrylique et d'hydrogène moléculaire sortant de l'évaporateur a été immédiatement envoyé, de bas en haut, à travers le réacteur tubulaire à double paroi. L'extrémité de ce dernier est à la pression atmosphérique. La température au milieu du réacteur est d'environ 220°C. Dans un séparateur à 10°C, on a séparé par condensation l'acide acrylique n'ayant pas réagi ainsi que l'acide propionique produit qui est contenu dans le courant de gaz produit.

Le condensat contient au bout de 813 g d'acide propionique d'une durée d'exploitation de 100h.

### Fabrication du propionate de vinyle

On réalise ensuite la réaction d'acyloxylation de l'éthylène sur l'acide propionique en phase gazeuse, dans un réacteur à lit fixe, au moyen d'un catalyseur au palladium sur un support en alumine, à une température de 180°C. On obtient en fin de réaction du propionate de vinyle obtenu à partir de carbone renouvelable.

## Revendications

1. Procédé de fabrication de propionate de vinyle comprenant les étapes suivantes :
a) fermentation de matières premières renouvelables et, éventuellement purification pour produire au moins un alcool choisi parmi l'éthanol et les mélanges d'alcools comprenant de l'éthanol ;
b) déshydratation de l'alcool obtenu pour produire, dans un premier réacteur, au moins un alcène choisi parmi l'éthylène et les mélanges d'alcènes comprenant de l'éthylène et, éventuellement purification de l'éthylène ;
c) production d'acide acrylique à partir de matières premières renouvelables,
d) hydrogénation de l'acide acrylique en présence d'hydrogène moléculaire pour produire de l'acide propanoïque,
e) introduction dans un troisième réacteur de l'éthylène obtenu à l'issue de l'étape b) et de l'acide propanoïque obtenu à l'issus de l'étape d), et mise en oeuvre de la réaction de acyloxylation de l'éthylène,
f) isolation et éventuellement purification du propionate de vinyle obtenu à l'issue de l'étape e).

2. Procédé de fabrication selon la revendication 1 dans laquelle l'étape c) est une déshydratation de glycérol en acroléine et oxydation de l'acroléine en présence d'oxygène moléculaire pour produire de l'acide acrylique.

3. Procédé de fabrication du propionate de vinyle selon la revendication 1 ou 2 dans laquelle l'étape c) est une oxydéshydratation de glycérol pour former l'acide acrylique.

4. Procédé de fabrication du propionate de vinyle selon la revendication 1 dans laquelle l'étape c) est réalisée par fermentation de matières premières renouvelables, pour produire de l'acide 3-hydroxypropionique, éventuellement purification, suivie d'une déshydratation de l'acide 3-hydroxypropionique en acide acrylique, éventuellement en présence d'oxygène moléculaire.

5. Procédé de fabrication selon l'une des revendications 1 à 4 **caractérisé en ce que** les matières premières renouvelables utilisées dans l'étape a) sont des matières végétales choisies parmi la canne à sucre et la betterave sucrière, l'érable, le palmier-dattier, le palmier à sucre, le sorgho, l'agave américain, le maïs, le blé, l'orge, le froment, le riz, la pomme de terre, le manioc, la patate douce, les algues.

6. Procédé de fabrication selon l'une des revendications 1 à 5, **caractérisé en ce que** la déshydratation de l'alcool est effectuée à l'aide d'un catalyseur à base d'alumine, de préférence à l'aide d'un catalyseur à base de gamma-alumine.

7. Procédé de fabrication selon l'une des revendications 1 à 6, **caractérisé en ce que** l'acyloxylation est effectuée en phase gazeuse, dans un réacteur à lit fixe, au moyen d'un catalyseur au palladium ou à l'acétate de palladium sur un support qui peut être du SiO₂ ou du Al₂O₃, à une température allant généralement de 175 à 200°C.

8. Procédé de fabrication selon l'une des revendications 1 à 7 **caractérisé en ce qu'**au moins une étape de purification est effectuée lors de l'étape a) et/ou b) et/ou c) et/ou d).

## Patentansprüche

1. Verfahren zur Herstellung von Vinylpropionat, das folgende Schritte umfasst:
a) Fermentation von nachwachsenden Rohstoffen und gegebenenfalls Reinigung zur Herstellung von mindestens einem Alkohol, der aus Ethanol und ethanolhaltigen Alkoholgemischen ausgewählt ist;
b) Dehydratisierung des erhaltenen Alkohols zur Herstellung von mindestens einem Alken, das aus Ethylen und ethylenhaltigen Alkengemischen ausgewählt ist, in einem ersten Reaktor und gegebenenfalls Reinigung des Ethylens;
c) Herstellung von Acrylsäure aus nachwachsenden Rohstoffen;
d) Hydrierung der Acrylsäure in Gegenwart von molekularem Wasserstoff zur Herstellung von Propansäure;
e) Eintragen des am Ende von Schritt b) erhaltenen Ethylens und der am Ende von Schritt d) erhaltenen Propansäure in einem dritten Reaktor und Durchführung der Acyloxylierungs-reaktion des Ethylens,
f) Isolierung und gegebenenfalls Reinigung des am Ende von Schritt e) erhaltenen Vinylpropionats.

2. Herstellungsverfahren nach Anspruch 1, bei dem es sich bei dem Schritt c) um eine Dehydratisierung von Glycerin zu Acrolein und Oxidation von Acrolein in Gegenwart von molekularem Sauerstoff zur Herstellung von Acrylsäure handelt.

3. Verfahren zur Herstellung von Vinylpropionat nach Anspruch 1 oder 2, bei dem es sich bei dem Schritt c) um eine Oxydehydratisierung von Glycerin zur Bildung von Acrylsäure handelt.

4. Verfahren zur Herstellung von Vinylpropionat nach Anspruch 1, bei dem der Schritt c) durch Fermentation von nachwachsenden Rohstoffen zur Herstellung von 3-Hydroxypropionsäure, gegebenenfalls Reinigung und anschließende Dehydratisierung der 3-Hydroxypropionsäure zu Acrylsäure, gegebenenfalls in Gegenwart von molekularem Sauerstoff, durchgeführt wird.

5. Herstellungsverfahren nach einem der Ansprüche 1 bis 4, bei dem es sich bei den im Schritt a) verwendeten nachwachsenden Rohstoffen um pflanzliche Stoffe, ausgewählt aus Zuckerrohr und Zuckerrübe, Ahorn, Dattelpalme, Zuckerpalme, Sorghum, amerikanischer Agave, Mais, Weizen, Gerste, Reis, Kartoffel, Maniok, Süßkartoffel und Algen, handelt.

6. Herstellungsverfahren nach einem der Ansprüche 1 bis 5, **dadurch gekennzeichnet, dass** die Dehydratisierung des Alkohols mit Hilfe eines Katalysators auf Basis von Aluminiumoxid, vorzugsweise mit Hilfe eines Katalysators auf Basis von gamma-Aluminiumoxid, durchgeführt wird.

7. Herstellungsverfahren nach einem der Ansprüche 1 bis 6, **dadurch gekennzeichnet, dass** die Acyloxylierung in der Gasphase in einem Festbettreaktor an einem Palladium- oder Palladium-acetat-Katalysator auf einem Träger, bei dem es sich um SiO₂ oder Al₂O₃ handeln kann, bei einer Temperatur, die im Allgemeinen im Bereich von 175 bis 200°C liegt, durchgeführt wird.

8. Herstellungsverfahren nach einem der Ansprüche 1 bis 7, **dadurch gekennzeichnet, dass** während des Schritts a) und/oder b) und/oder c) und/oder d) ein Reinigungsschritt durchgeführt wird.

## Claims

1. A process for the manufacture of vinyl propionate comprising the following stages:
a) fermentation of renewable starting materials and optionally purification in order to produce at least one alcohol chosen from ethanol and mixtures of alcohols comprising ethanol;
b) dehydration of the alcohol obtained in order to produce, in a first reactor, at least one alkene chosen from ethylene and mixtures of alkenes comprising ethylene and optionally purification of the ethylene;
c) production of acrylic acid from renewable starting materials,
d) hydrogenation of the acrylic acid in the presence of molecular hydrogen in order to produce propanoic acid,
e) introduction, into a third reactor, of the ethylene obtained on conclusion of stage b) and of the propanoic acid obtained on conclusion of stage d), and implementation of the reaction for the acyloxylation of the ethylene,
f) isolation and optionally purification of the vinyl propionate obtained on conclusion of stage e).

2. The manufacturing process as claimed in claim 1, in which stage c) is a dehydration of glycerol to give acrolein and oxidation of the acrolein in the presence of molecular oxygen in order to produce acrylic acid.

3. The process for the manufacture of vinyl propionate as claimed in claim 1 or 2, in which stage c) is an oxydehydration of glycerol in order to form acrylic acid.

4. The process for the manufacture of vinyl propionate as claimed in claim 1, in which stage c) is carried out by fermentation of renewable starting materials, in order to produce 3-hydroxypropionic acid, optionally purification, followed by a dehydration of the 3-hydroxypropionic acid to give acrylic acid, optionally in the presence of molecular oxygen.

5. The manufacturing process as claimed in one of claims 1 to 4, **characterized in that** the renewable starting materials used in stage a) are plant materials chosen from sugarcane and sugar beet, maple, date palm, sugar palm, sorghum, maguey, corn, wheat, including soft wheat, barley, rice, potato, cassava, sweet potato, algae.

6. The manufacturing process as claimed in one of claims 1 to 5, **characterized in that** the dehydration of the alcohol is carried out using an alumina-based catalyst, preferably using a catalyst based on gamma-alumina.

7. The manufacturing process as claimed in one of claims 1 to 6, **characterized in that** the acyloxylation is carried out in the gas phase in a fixed bed reactor using a palladium or palladium acetate catalyst on a support which can be SiO₂ or Al₂O₃ at a temperature generally ranging from 175 to 200°C.

8. The manufacturing process as claimed in one of claims 1 to 7, **characterized in that** at least one purification stage is carried out during stage a) and/or b) and/or c) and/or d).
